# EUROPEAN PATENT APPLICATION

(11) **EP 1 398 320 A1**
(43) Date of publication of application: **17.03.2004**
(21) Application number: 03425573.7
(22) Date of filing: 05.09.2003
(51) Int. Cl.: C07J 71/00

(54) **Preparative separation of steroids by reverse phase HPLC**

(30) Priority: 13.09.2002 IT RM20020458
(71) Applicant: Ambiotec sas di Ammendola Sergio, 00040 Roma (IT)
(72) Inventor: Ammendola, Sergio, 00040 Rome (IT); Fabbri, Armando, E-00176 Rome (IT)

(57) **Abstract**

Budesonide C22R epimer is more active than C22S epimer. In so far the preparative separation of the two molecules from the mixture has not been made suitable at industrial level. The present invention describes the procedure to separate at preparative scale the epimers of certain synthetic steroid mixture. The present invention has based on isocratic reverse phase HPLC method and some devices as the procedure to dissolve the mixture and load it on the column, the choice of solvent and the physic-chemical condition of the chromatography, the recovery of the solvents. The whole of these devices to make the process to separate epimers from steroid mixtures, practicable at industrial level. More in particular the present invention describes the separation of the (22 R, S) 16α,17α-butyldenedioxy-11β, 21-dihydroxypregna-1,4-diene-3,20-dione (Budesonide), at preparative scale, into C22R and C22S epimers. The Budesonide has dissolved in a some organic solvent and each epimer has eluted in a water solution, permitting a highly easily recovery of the molecules also useful to pharmaceutical formulations.

## Description

### Field of the invention

The present invention relates to a chromatographic process to separate the R epimer from the S epimer of a certain steroid, starting from its stereoisomeric mixture; often epimers have a different pharmacological effect and the availability of the two separate drug may be an advantage.

More in particular the invention describes an unusual reversed-phase chromatography to separate the mixture (22 R, S) 16α,17α-butyldenedioxy-11β, 21-dihydroxypregna-1,4-diene-3,20-dione (budesonide), at preparative scale, into C22R and C22S epimers.

### Prior Art

Generally steroids have synthesized as stereoisomeric mixture which racemic or epimeric compounds often having a different pharmacological activity. Recently the mixture of pharmaceuticals have separated into components by several methods. Chromatographic may be a process to separate glucocorticoids as budesonide but in so far the only one preparative process by molecular exclusion chromatography (gel filtration) was described in the expired patent US3928326. The HPLC method on the sephadex LH20 describes a process to separate about 1500 mg of budesonide with a column of 6.3 cm x 75 cm by using a 20:20:1 mixture of epthan-chloroform-ethanol. The process uses a column having a volume of 2,3 litres to separate about 750 mg/l of budesonide mixture and it has been considered not suitable at industrial scale also tacking in account the high cost of the solvents. As a matter of the fact the patents WO 92/11280 from Instytut Farmaceutyczny, the Sicor SpA patents and Farmabios Srl patents (EP 0875516 A2, EP 09941991 Al) all describe methods to synthesize the C22R of budesonide and all stress that the gel filtration chromatographic method have technologically inconvenient, expensive and time-consuming. Moreover the C22R has obtained with a purity ranging among 90%-96%.

However C22S epimer is pharmacological active and the patented syntheses failure to one step recovery of both epimer molecules.

On the other side analytical separation of budesonide have described and the use of reversed phase C18 has been successful used. The European Pharmacopoeia describes a reverse phase HPLC method that separates epimer C22R from C22S. However several analytical chromatographic methods have been described (Wikby et al. 1978, J. chromatog., Roth G. et al. 1980, J. Pharm. Sci. 69,766-770; Shuguang H. et al. 2001, J. Pharm. Biom. Anal. 24,371-380).

In so far all described methods, differing in the solvents, fail at preparative scale due to the relative solubility and lipophilicity of the budesonide and solvent viscosity. In fact has been described the higher budesonide viscosity in certain mobile phase and lower solute diffusion coefficients causes greater plates heights. The results are that all classical methods fail to separate the budesonide epimers at high concentration.

Although it is not clarified the use of a suitable water: solvent solution has been proposed to be important in the chromatographic behaviour of budesonide epimers. On the basis of the possibility to dissolve budesonide in alcohol, but utilizing a not obvious method, we set up a process to separate budesonide epimers at preparative scale.

### Detailed description of preferred embodiments

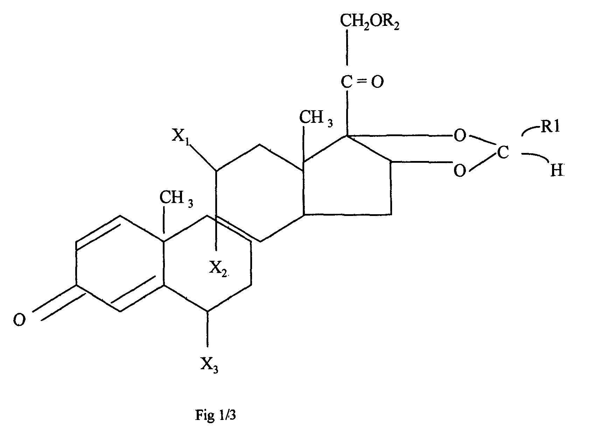

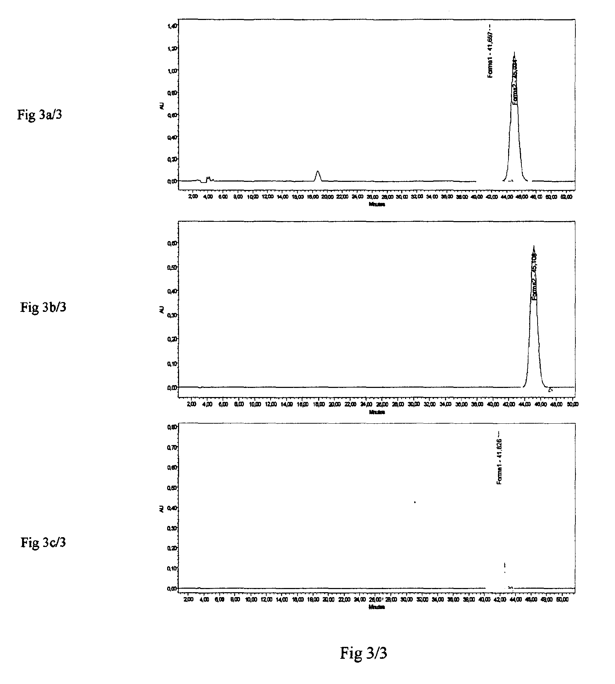

A HPLC chromatographic process to purify synthetic steroids and when occurring, to separate stereoisomeric mixture in their component, has described herein. The present invention may be suitable for purification at preparative scale of synthesized glucocorticoids as showed in FIG 1/3 and their derived or chemically related molecules.

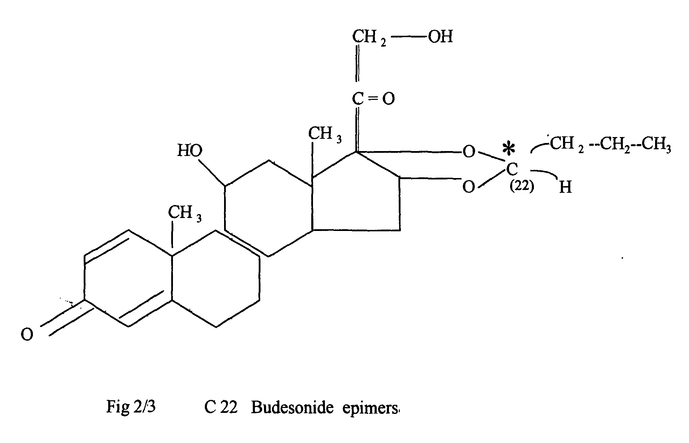

More in particular the process is suitable to separation of (22 R, S) 16α,17α-butyldenedioxy-11β,21-dihydroxypregna-1,4-diene-3,20-dione and related molecules. Budesonide (FIG 2/3) is a racemate consisting of mixture of two epimers, C22R and C22S, generally present in a non-equimolar ratio.

Furthermore acting on solvents, loading conditions, elution and geometry of column, it is possible purify not only 16α,17α-butyldenedioxy-11β,21-dihydroxypregna-1,4-diene-3,20-dione derived molecules but also steroids as well as progesterone and ursodeoxycholic acid.

The process described in the present invention consists of a method to optimize relationship among budesonide solubility, viscosity and lipophilicity. In the present invention has described a HPLC isocratic method to purify steroids and separate glucocorticoid epimers where the stationary phase is a C18 and if required an ion-pair reagent can be used.

In this process, where viscosity and lipophilicity are strategic to optimize separation, each chromatography must be carried on one-step cycle and in this view the use of solvent maximizing the concentration of loaded substance, has preferred. The ratio between the loading volume (V_{L}) and the volume of the column (V_{C}) must be at least 0.02 (V_{L}/ V_{C} ≤ 0.02). With this process it is possible separate the epimers at a purity of 98% and a recovery more than 95%. The functional group C18 may be choice having granulometry ranging among 3,5 µm and 15 µm.

### Detailed description of drawings

**FIG 1/3** represents a glucocorticoid where R1 may be a linear or branched alkyl having among 1 up to 10 carbon atoms. X1 generally is a hydroxyl and X2 and X3 are hydrogen or phluoro atoms or a halogen, R2 may be and hydroxyl, an ester or an acylic group.

(22 R, S) 16α,17α-butyldenedioxy-11β,21-dihydroxypregna-1,4-diene-3,20-dione (budesonide).

**FIG 2/3** is a particular case where (22 R, S) 16α,17α-butyldenedioxy-11β, 21-dihydroxypregna-1,4-diene-3,20-dione (budesonide).

**FIG 3/3** (3a/3, 3b/3, 3c/3) are the chromatograms of budesonide on the C18 column according to the present process (3a), and the epimer S after the process (3b) and the epimer R after the process (3c).

### Example 1

In a preferred process to separate C22 (R,S) budesonide epimers, the drug mixture (purchased by SIGMA and ASTRA-ZENECA) must be dissolved in methylic alcohol and at a concentration ranging between 33 g/l and 40 g/l and more, loaded on the column Inertisil ODS 3 column (4.6 mm x 250 mm, 5 micron) to reach the best purity. The column has washed with a bufierA (acetonitrile:water 30:70) at a flux of 1 ml/min and 1500 psi. After the budesonide mixture, carefully dissolved, has loaded on the column and the epimers separation has obtained washing with the bufferA. Each epimer has followed at 240 nm wave length.

The eluted fraction have analysed according our analytical method obtained loading about 4 mg of budesonide on the C18 column and recovering two fractions of about 7.5 ml, respectively (FIG 3a/3). The fraction 1 (R epimer) having a concentration of 0.283 mg/ml (FIG 3b/3) and the fraction 2 (S epimer) having a concentration of 0.253 mg/ml (FIG 3c/3).

A budesonide standard has been used to titre spectrophotometrically the yield of the process; a calibration curve has been constructed at 240 nm among 8 µg/ml up to 70 µg/ml.

**RESULTS**

| **Fraction** | **Volume** | **U.V. Concentration** | **yield** | **recovery*** | **Percentage of purity** |
|---|---|---|---|---|---|
| | ml | mg/ml | mg | % | % |
| **load** | 0.1 | 40.28 | 4.028 | | |
| **R epimer** | 7.5 | 0.283 | 2.12 | 52.6 | >98 |
| **S epimer** | 7.5 | 0.253 | 1.897 | 47.1 | >98 |
| Where in this example the epimers composition is: R epimer = 52.45% S epimer = 47.55%. | | | | | |
| Yield of the process is R-epimer = **100.3%** S- epimer = **99%** | | | | | |

### Example 2

A process to purify certain steroids according to example 1 method but in presence of trifluoracetic acid or phosphoric acid or acetic acid (at the final concentration of 0.1%). A shift of the retation time as well the use of a ion pair reagent may result suitable to purify molecule from impurities or to separate epimers from pure mixtures.

### Example 3

According to the procedure of the preferred example 1,2.3 g of budesonide mixture have loaded on a C18 column with the following parameters
Column geometry: 110 mm x 250 mm
Column volume or phase : 2374 ml
Flux: 571 ml/min
Loading volume: 60 ml
Loading mass: 2400 mg

### Example 4

According to the present invention it is possible change some important parameters which are the body of the present invention to use stationary phases having the same or equivalent C18 functional group but other matrix granulometry, poresize, capping chemistry, density of linking group. As a matter of fact it is possible a preparative purification of steroids and a preparative separation of epimers by using the following columns as stationary phase: X-terra 3.5 µm (Waters); ODS3 inertsil 5 µm (GL-science); Daisogel ODS-AP SP120 (Daiso) 10 µm where the support is Silica gel or Resource Rp (Pharmacia) 15 µm where the support is "polystyrene"/divynilbenzene or some other stationary phase suitable for the reverse-phase. As a matter of fact according to the present invention it is possible that the system to purify and to separate the progesterone or ursodeoxycholic acid may not be the optimum for all steroids and it can be set up aging on ion pair reagent and acetonitrile:water ratio, respectively.

### Example 5

According to present invention it is possible a preparative purification of the ursodeoxycholic acid dissolving in methanol at the concentration of 100 g/l. About 6g have loaded on the ODS3 column Inertisil 5 micron (GL sciences Inc.). In this example the best mobile phase is the acetonitrile:water 40:60 where the process reach a molecules purity of the 99.3% and a recovery of the 97.8%.
Column geometry: 110 mm x 250 mm
Phase volume: 2374
Flux: 571 ml/min
Loading volume: 60 ml
Loading mass: 6 g

On the basis of the present invention it is possile to scale-up the process according to our result the quality of separation do not change loading more steroid mass. The available Inertsil ODS3 Prep 5 micron give the following results:

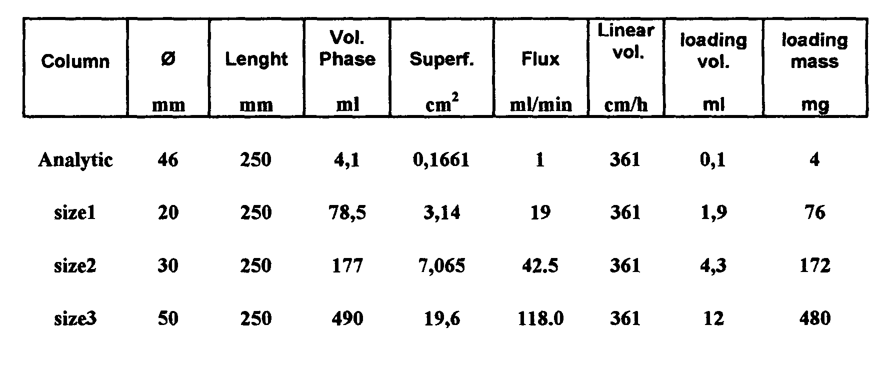

## Claims

1. a chromatographic procedure to preparative purification of synthetic steroids, including epimers separation, where it use a reverse phase under isocratic condition. The functional group is a any C18 or its equivalent binding group immobilized on silice or "polystyrene"/divynilbenzene having a granulometry ranging among 3.5 µm up to 15 µm.

2. a chromatographic method according to claim 1. where the mobile phase is acetronitrile:water in the preferred ratio 30:70 and where the ion pair reagent can be choiced among trifluoracetic acid, acetic cid or phosphoric acid.

3. A method according to claims 1. and 2., where the mixture of epimers is dissolved at the max concentration by using a suitable solvent and after it has loaded on the column without perturbing the composition of the mobile phase.

4. A method according to claims 1., 2., and 3. where the minimum ratio between the loading volume and the volume of the phase is 0.02 with a resolution among 99% and 97% and the recovery of the molecules is among 96% and 90%.

5. A method according to claims 1., 2., 3., and 4., where the steroid is the budesonide dissolved at the minimal concentration of 40 g/litre in the methanol as preferred solvent.
